Europäisches Patentamt

European Patent Office

Office européen des brevets

(11) Publication number : **0 485 118 A1**

## (12) EUROPEAN PATENT APPLICATION

(21) Application number : **91310093.9**

(22) Date of filing : **31.10.91**

(51) Int. Cl.⁵ : **A61B 5/00**

(30) Priority : **08.11.90 US 612113**

(43) Date of publication of application :
**13.05.92 Bulletin 92/20**

(84) Designated Contracting States :
**AT BE CH DE DK ES FR GB GR IT LI LU NL SE**

(71) Applicant : **PURITAN-BENNETT CORPORATION**
**9401 Indian Creek Parkway, Overland Park**
**Kansas City, Kansas (US)**

(72) Inventor : **Riccitelli, Samuel D.**
**2378 Terraza Ribera**
**Carlsbad, California 92009 (US)**
Inventor : **Divers III, George A.**
**12292 Escala Drive**
**San Diego, California 92128 (US)**

(74) Representative : **Mayes, Stuart David et al**
**BOULT, WADE & TENNANT 27 Furnival Street**
**London, EC4A 1PQ (GB)**

(54) Intravascular blood parameter sensor apparatus.

(57)    The intravascular blood parameter sensor apparatus (10) includes an elongated introducer catheter (12) and an elongated sensing catheter (30) having a sensor region (34) to be extended through the introducer catheter (12) into the vasculature of the patient. At least one blood parameter sensor (36) is disposed within the sensor region (34) of the sensing catheter (30). The blood parameter sensor apparatus (10) is positioned within a blood vessel (22) of a patient by inserting the introducer catheter (12) into the blood vessel (22) at an angle relative to the blood vessel (22) for a sufficient distance that the distal portion (18) lies essentially parallel to the axis of the blood vessel (22), and extending the sensing catheter (30) through the distal opening (20) of the introducer catheter (12) so that the distal sensor region (34) extends beyond the distal opening (20) of the introducer catheter (12).

FIG. 1

This invention is generally directed to chemical and biochemical analysis for an analyte in a fluid or gaseous mixture, and more specifically concerns the intravascular placement of blood parameter sensing devices which monitor in vivo blood oxygen levels.

As more sophisticated life support systems have become available, the measurement of the tension or partial pressure of oxygen and carbon dioxide in blood, and the acidity of blood, have become increasingly important. These measurements are particularly useful in determining the respiratory status of a patient on a respirator. Blood parameter sensor probes utilizing optical fibers have been developed which are small enough to be inserted into a blood vessel of a patient through a small catheter. Such a sensor can be placed in the blood vessel for a relatively long period of time without otherwise disturbing the patient, thus providing the capability to continuously monitor the patient's blood chemistry. A slow heparin flush can be maintained through the introducer catheter to prevent coagulation on the probe, until the probe is removed. Blood pressure may also be measured directly through the introducer catheter.

When such a sensor probe according to the prior art is inserted into a patient's vasculature, the sensor on the distal tip of the elongated fiber optic probe may be forced into the intravascular endothelial tissue. It has been found that the intravascular tissue may possess a different concentration of an analyte, such as oxygen, from that in the moving blood stream. In one prior art blood gas sensor probe which attempts to address this problem, the blood gas sensor is contained within an open-ended catheter that is kept free of blood clots by a slow flow of an anti-clotting solution through the catheter and around the sensor bundle. While this may be effective in preventing insertion of the probe into the endothelial wall, such an approach can interfere with the effective operation of the sensor and the monitoring of blood pressure through the catheter.

It would be desirable for the sensor placement catheter to have a configuration which could cause the sensor to rest upon or away from the surface of the vascular tissue to insure that measurements of a blood analyte of interest are not biased by the concentration of the analyte in the vascular tissue, and to help reduce the possibility of injury to the vascular tissue. It would also be desirable to reduce possible interference by flushing of a substantial quantity of an anti-clotting solution with the operation of the sensor, and to provide a configuration which will allow the effective monitoring of blood pressure through an introducing catheter.

According to a first aspect of the present invention, there is provided an intravascular blood parameter sensor apparatus adapted to be inserted into a blood vessel of a patient, comprising:

a flexible elongated introducer catheter adapted to be inserted into the blood vessel, the blood vessel having a longitudinal axis, the catheter having a longitudinal axis and a distal opening adapted to extend a distance into the blood vessel sufficient for the axis of the catheter at the distal opening to extend essentially parallel to the longitudinal axis of the blood vessel;

an elongated probe member having a longitudinal axis and a distal sensor region adapted to extend through the introducer catheter beyond the distal opening of the introducer catheter into the blood vessel a distance sufficient to substantially minimize an angle formed between the axis of the probe member and the axis of the blood vessel; and

at least one blood parameter sensor disposed within the sensor region of the probe member.

The introducer catheter is preferably long enough to extend into the blood vessel a sufficient distance so that the mouth of the introducer catheter is substantially perpendicular to the blood vessel, and the probe member is preferably long enough so that, when extended into the introducer catheter, the sensor region of the probe will rest upon, or away from, the blood vessel wall.

According to a second aspect of the present invention, there is provided a method of positioning a blood parameter sensor apparatus within a blood vessel of a patient, the apparatus including an elongated probe member having a distal end adapted to be inserted through an introducer catheter into the blood vessel, said probe member having a distal sensor region, the method comprising the steps of:

inserting said introducer catheter into said blood vessel at an angle relative to the longitudinal axis of said blood vessel; and

extending the distal end of the probe member to curve and extend parallel to the blood vessel beyond the distal opening of the introducer catheter at least 0.5 cm beyond the distal opening of the introducer catheter.

The introducer catheter is preferably inserted into the blood vessel at an oblique angle relative to the blood vessel a distance sufficient to place the distal portion of the introducer catheter approximately parallel with the wall of the blood vessel; and the distal sensor region of the probe member is preferably inserted through the distal opening of the introducer catheter until the sensor region extends a distance beyond the distal opening of the introducer catheter sufficient to prevent any substantial angularity between the distal axis of the probe and the vessel wall.

An embodiment of the present invention will now be described, by way of example, with reference to the accompanying drawing which shows an intravascular blood parameter sensor in accordance with the present invention disposed within a blood vessel.

The intravascular blood parameter sensor shown in the accompanying drawing comprises an elongated, tubular sensing catheter member which is adapted to be introduced through an introducer catheter into a blood vessel of a patient. The sensing catheter includes a distal sensor region which extends 0.5 cm or more beyond the distal opening of the introducer catheter, to avoid the phenomena of interference by the tissue at the inner wall of the blood vessel and anti-coagulant solution with measurements made by the sensor. The intravascular introducing catheter is preferably long enough so that the distal end of the introducing catheter can extend a sufficient distance into a blood vessel to help insure that the distal portion of the introducer catheter will be positioned approximately parallel to the vascular wall, so that the probe member will exit the introducer with a minimum degree of angularity relative to the blood vessel wall, thus allowing the sensor probe to rest on, or away from, the blood vessel wall.

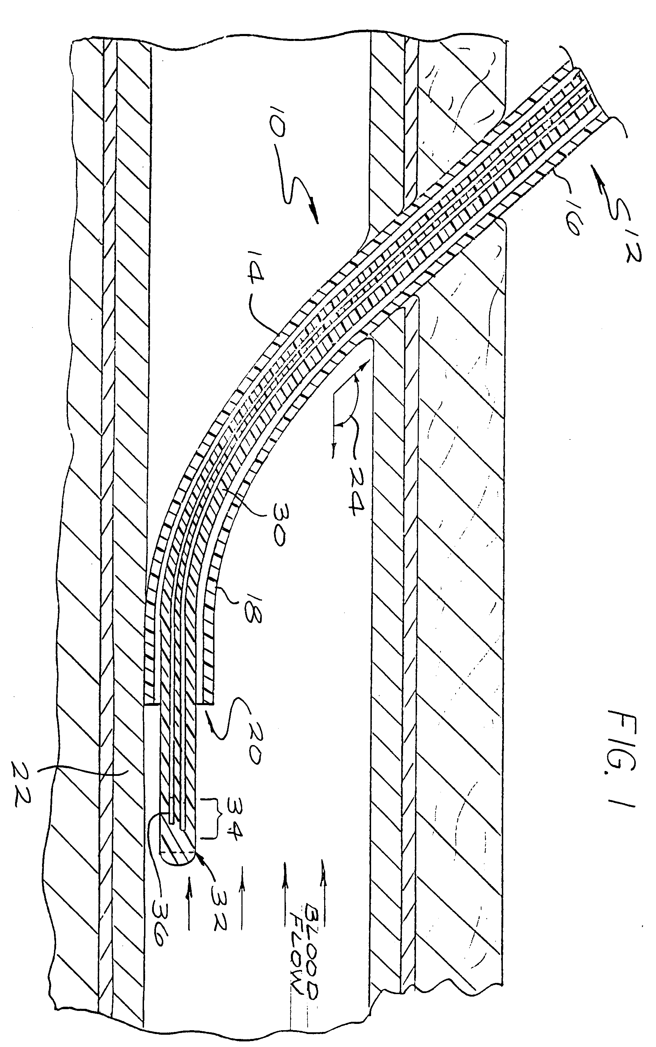

With reference particularly to Figure 1 of the drawings, the intravascular blood parameter sensor probe apparatus 10 includes flexible, elongated, introducer catheter 12, having a tubular shaft 14 with a relatively straight proximal portion 16 and a distal portion 18 ending in a distal opening 20. The introducer catheter is preferably adapted to be inserted into a blood vessel 22 of a patient at an oblique angle 24 relative to the blood vessel. The introducer catheter is preferably long enough to extend into the blood vessel a sufficient distance to allow the distal portion of the introducer catheter to assure a position essentially parallel to the vessel wall. In a preferred embodiment of the invention, the introducing catheter is at least one and one quarter inches (1-1/4″) long, and can advantageously be approximately 1.75 to 2 inches long for insertion into the blood vessel of an adult patient. This length helps insure that the distal portion of the introducer catheter is approximately parallel to the longitudinal axis of the blood vessel, and that the sensing catheter will exit the introducer with a minimum degree of angularity relative to the blood vessel wall thereby allowing the sensor probe to rest on, or away from, the blood vessel wall. The distal portion of the introducer catheter preferably flexes to curve at an oblique angle relative to the proximal shaft portion of the introducer, and thereafter extends distally so as to be generally parallel to the blood vessel wall when the introducer catheter is inserted into a patient's blood vessel.

An elongated tubular sensing catheter or probe member 30, which is adapted to be inserted through the introducer catheter, has a distal end 32 to be extended into the blood vessel, and includes a distal sensor region 34 which extends beyond the distal opening of the introducer catheter. In a preferred embodiment, the distal sensor region extends at least 0.5 cm or more beyond the distal opening of the introducer catheter. By this combination of the configuration of the introducer catheter and the sensing catheter, the sensing catheter is not exposed to interference by concentrations of the analyte of interest in the tissue of the inner wall of the blood vessel that are different from those of the blood in the vessel. The sensing catheter includes at least one blood parameter sensor 36, such as an oxygen, carbon dioxide, blood pH, or temperature sensor, disposed within the sensor region of the sensing catheter. The sensing catheter is further configured so that no individual sensor within the sensing catheter will be less than the minimum distance of 0.5 cm from the distal end of the introducer, which has been found to be effective to produce accurate readings by the sensor. This also insures that if a slow flow of an anticoagulant solution is provided through the introducer catheter to reduce thrombogenesis, the anti-coagulant solution will be sufficiently dispersed before it reaches the sensor region so that it will not interfere with the measurements of the sensing catheter. In a currently preferred embodiment, an optimum minimum distance has been found to be approximately 3 cm. When the probe is inserted into the vasculature of the patient, this minimum distance has been found to reduce the force with which the sensing catheter will press on the blood vessel wall as it emerges from the distal opening of the introducer catheter and extends along the vascular wall.

The preferred method of intravascular positioning of the blood parameter sensor apparatus involves first inserting the introducer catheter into the blood vessel at an angle relative to the longitudinal axis of the blood vessel, which is most preferably an oblique angle relative to the axis of the blood vessel. The introducer catheter should be inserted a distance sufficient to place the distal portion of the introducer catheter approximately parallel with the wall of the blood vessel, so that the mouth of the introducer catheter is substantially perpendicular to the blood vessel.

The probe member is also preferably inserted into the patient's blood vessel through the proximal end of the introducer catheter until the sensor region extends a distance beyond the distal opening of the introducer catheter sufficient to prevent any substantial angularity between the distal axis of the probe and the vessel wall so that the sensor region of the probe will rest upon, or away from, the blood vessel wall. The probe sensor region is currently typically extended at least about 0.5 cm and not more than about 3 cm beyond the distal opening in said introducer catheter.

Thus it will be apparent to those skilled in the art that the embodiment described provides for proper placement of a blood parameter sensing device which reduces the possibility of penetration of the inner wall of a blood vessel and the attendant interference with intravascular in vivo measurement of the blood parameter. In particular, the embodiment provides means to assure that a blood analyte sensing catheter probe will not become partially imbedded in vascular tissue, such as the endothelial tissue on the inner wall of an artery. The sensing catheter and introducer structure of the embodiment provide this benefit by guiding the sensing catheter so that the sensor region of the sensing catheter is not forced into the vascular tissue, but merely rests on, or away from the inner surface of the vascular tissue.

**Claims**

1. An intravascular blood parameter sensor apparatus adapted to be inserted into a blood vessel of a patient, comprising:

   a flexible elongated introducer catheter adapted to be inserted into the blood vessel, the blood vessel having a longitudinal axis, the catheter having a longitudinal axis and a distal opening adapted to extend a distance into the blood vessel sufficient for the axis of the catheter at the distal opening to extend essentially parallel to the longitudinal axis of the blood vessel;

   an elongated probe member having a longitudinal axis and a distal sensor region adapted to extend through the introducer catheter beyond the distal opening of the introducer catheter into the blood vessel a distance sufficient to substantially minimize an angle formed between the axis of the probe member and the axis of the blood vessel; and

   at least one blood parameter sensor disposed within the sensor region of the probe member.

2. The apparatus of Claim 1, wherein the introducer catheter is from about 1.25 inches to about 2 inches long.

3. The apparatus of Claim 1 or 2, wherein the distal portion of said probe member extends at least 0.5 centimeters beyond the distal opening of said introducer catheter.

4. The apparatus of claim 1, 2 or 3, wherein the distal portion of said probe member extends from about 0.5 cm to about 3 cm beyond the distal opening of said introducer catheter.

5. A method of positioning a blood parameter sensor apparatus within a blood vessel of a patient, the apparatus including an elongated probe member having a distal end adapted to be inserted through an introducer catheter into the blood vessel, said probe member having a distal sensor region, the method comprising the steps of:

   inserting said introducer catheter into said blood vessel at an angle relative to the longitudinal axis of said blood vessel; and

   extending the distal end of the probe member to curve and extend parallel to the blood vessel beyond the distal opening of the introducer catheter at least 0.5 cm beyond the distal opening of the introducer catheter.

6. The method of Claim 5 further comprising the step of:

   inserting the introducer catheter at an angle to and for a distance into said blood vessel sufficient to place the distal end of said introducer catheter essentially parallel to the axis of said blood vessel.

7. The method of Claim 5 or 6, further comprising the step of extending the distal end of said probe member beyond the distal opening of the introducer catheter.

8. The method of Claim 7, wherein said distal end of said probe member is extended at least 0.5 cm beyond the distal opening of the introducer catheter.

9. The method of Claim 7, wherein said distal end of said probe member is extended from about 0.5 cm to about 3 cm beyond the distal opening of the introducer catheter.

FIG. 1

**European Patent Office**

**EUROPEAN SEARCH REPORT**

Application Number

EP    91 31 0093

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-3 983 864 (SIELAFF ET AL.)<br>* column 3, line 62 - column 4, line 46; figures 1,2A * | 1,5 | A61B5/00 |
| A | | 3,6-8 | |
| | --- | | |
| Y | EP-A-0 307 162 (C. R. BARD, INC.)<br>* column 3, line 56 - column 6, line 20 *<br>* column 7, line 9 - column 9, line 12 *<br>* figures 1,2,5,6 * | 1,5 | |
| | --- | | |
| A | ANALYTICAL CHEMISTRY.<br>vol. 61, no. 21, 1 November 1989, COLUMBUS US<br>pages 2365 - 2372;<br>COLLISON ET AL.: 'Potentiometric combination ion/carbon dioxide sensors for in vitro and in vivo blood measurements.'<br>* page 2365, left column, line 30 - page 2366, right column, line 41 *<br>* page 2369, left column, line 16 - line 36 *<br>* figures 1,4 * | 1-5,7-9 | |
| | --- | | TECHNICAL FIELDS SEARCHED (Int. Cl.5 ) |
| A | US-A-3 878 830 (BICHER)<br>* column 3, line 61 - column 4, line 47 *<br>* column 6, line 61 - column 7, line 12 *<br>* figures 1,2 * | 1,5 | A61B |
| | --- | | |
| P,X | EP-A-0 442 276 (THE BOC GROUP, INC.)<br>* abstract *<br>* page 6, line 11 - line 26; figure 1 * | 1,5 | |
| | ----- | | |

The present search report has been drawn up for all claims

| Place of search | Date of completion of the search | Examiner |
|---|---|---|
| THE HAGUE | 30 JANUARY 1992 | CHEN A.H. |